# EUROPÄISCHE PATENTSCHRIFT

(11) **EP 1 907 386 B1**
(45) Veröffentlichungstag und Bekanntmachung des Hinweises auf die Patenterteilung: **02.06.2010**
(21) Anmeldenummer: 06762730.7
(22) Anmeldetag: 20.07.2006
(51) Int. Cl.: C07D 417/12, A61K 31/435, A61P 3/00

(54) **AMINOSÄURESALZE VON ROSIGLITAZON**
AMINO ACID SALTS OF ROSIGLITAZONE
SELS D'ACIDES AMINES DE ROSIGLITAZONE

(30) Priorität: 22.07.2005 DE 102005034406
(43) Veröffentlichungstag der Anmeldung: 09.04.2008
(73) Patentinhaber: Ratiopharm GmbH, 89079 Ulm (DE)
(72) Erfinder: WERZ, Udo, 88480 Achstetten-Stetten (DE); MAAS, Gerhard, 89250 Senden (DE); STAHL, Heinrich, 79104 Freiburg (DE)
(74) Vertreter: Best, Michael
(86) Internationale Anmeldenummer: PCT/EP2006/007171
(87) Internationale Veröffentlichungsnummer: WO 2007/009799

(56) Entgegenhaltungen:
- WO-A-01/94343
- WO-A-01/94344
- WO-A-02/12232
- WO-A-02/20517
- WO-A-02/20519
- WO-A-02/20520
- WO-A-94/05659
- WO-A-02/051839
- WO-A-03/045946
- WO-A-03/045947
- WO-A-03/050111
- WO-A-03/050112
- WO-A-03/050113
- WO-A-03/050115
- WO-A-03/050116
- WO-A-03/053962
- WO-A-03/053963
- WO-A-03/053964
- WO-A-03/053965
- WO-A-2005/023803

## Beschreibung

Die vorliegende Erfindung betrifft neue Salze von Rosiglitazon, nämlich Aminosäuresalze von Rosiglitazon und deren Solvate, Arzneimittel, die solche Salze oder Solvate enthalten, deren Verwendung für die Behandlung von bestimmten Krankheiten, sowie Verfahren zur Herstellung dieser Salze. Insbesondere betrifft die Erfindung das Cholinat, das Lysinat und das Arginat der racemischen oder einer enantiomeren oder tautomeren Form von Rosiglitazon, wobei das Cholinat auch wegen seiner guten Wasserlöslichkeit besonders bevorzugt ist.

Rosiglitazon ist die INN-Bezeichnung für 5-(4-(2-(N-methyl-N-(2-pyridyl)amino)ethoxy)benzyl)-2,4-thiazotidinedion und wird in der EP 0 306 228 B1 ausführlich beschrieben. Es eignet sich für die Behandlung und Prophylaxe von Hyperglykämie, insbesondere Typ II Diabetes, Hyperlipämie, Hochdruck, kardiovaskulären Erkrankungen und bestimmten Essstörungen. Das Maleatsalz soll besser löslich sein als die freie Base, gute Stabilität besitzen sowie aufgrund seiner verbesserten Selektivität insbesondere gegen Typ II Diabetes einsetzbar sein. Es wird in der EP 0 658 161 B1 beschrieben. In der WO94/05659 wird darüber hinaus das Tartratsalz offenbart. Die WO02/12232 offenbart das DL-Tartrat, welches sich von dem D-Tartrat und dem L-Tartrat unterscheiden und vorteilhafte Eigenschaften besitzen soll. Das Hydrochlorid-Salz von Rosiglitazon ist Gegenstand der WO02/20519 und das Phosphat-Salz wird in der WO05/023803 offenbart. Es soll eine hohe Wasserlöslichkeit aufweisen, die aber durchaus noch nicht zufriedenstellend ist.

WO 03/045946 A1 offenbart das Benzolsulfonatsalz des Rosiglitazons, und Solvate davon.

WO 021051839 A1 offenbart das Rosiglitazonsalz der Methansulfonsäure der Form I bis IV. und Solvate davon.

WO 02/20520 A1 offenbart das Rosiglitazonsalz der Ethansulfonsäure, und ein Solvat davon.

WO 02/20517 A1 offenbart das Rosiglitazonsalz der Fumarsäure und ein Solvat davon.

WO 01/94343 A1 offenbart das Rosiglitazonsalz der Jodwasserstoffsäure und ein Solvat davon.

WO 01/94344 A1 offenbart das Rosiglitazonsalz der Bromwasserstoffsäure und ein Solvat davon.

WO 03/045947 offenbart das Rosiglitazonsalz 1,2-Ethandisulfonsäure.

WO 03/053965 A1 offenbart das Rosiglitazonsalz der Salicylsäure.

WO 03/053964 A1 offenbart das Rosiglitazonsalz der 2-Naphthylsufonsäure.

WO 03/053963 A1 offenbart das Rosiglitazonsalz der 1-Hydroxy-2-Naphthylsäure.

WO 03/053962 A1 offenbart das Rosiglitazonsalz der Äpfelsäure.

WO 03/050116 A1 offenbart das Rosiglitazonsalz der 10-Camphorsulfonsäure.

WO 03/050115 A1 offenbart das Rosiglitatzonsalz der Zitronensäure.

WO 03/050113 A1 offenbart das Hydrogensulfatsalz des Rosiglitazons.

WO 031050112 A1 offenbart ein p-Toluolsulfonsäurehydratsalz des Rosiglitazons.

WO 03/050111 A1 offenbart das p-Toluolsulfonat des Rosiglitazons.

Es besteht also ein Bedürfnis nach neuen Salzen des Rosiglitazons, die dessen Verwendungsmöglichkeiten erweitern. Die neuen Salze sollen insbesondere eine gute Löslichkeit, insbesondere unter physiologischen Bedingungen aufweisen.

Ein Kriterium für die Einsatzmöglichkeiten neuer Salze des Rosiglitazons ist dabei, dass durch das pharmazeutisch nicht aktive Anion, das gewisse Sekundäreigenschaften der pharmazeutisch aktiven Base verändern soll, nicht Substanzen mit möglicherweise nachteiligen oder sogar schädlichen Eigenschaften in den Körper eingebracht werden. Das Anion soll also nicht körperfremd sein, möglichst eine Substanz, die ohnehin im Körper vorhanden ist oder deren Zufuhr möglicherweise sogar vorteilhaft ist.

Es wurde nun gefunden, dass die Aminosäuren sich für die Salzbildung des Rosiglitazons vorteilhaft eignen. Die Aminosäuren sind teilweise sogar essentielle Bestandteile des Körpers, also keine körperfremden Stoffe, ihre Zufuhr ist vielfach sogar gewünscht. Die erfindungsgemäßen Aminosäuresalze zeichnen sich also durch gute Verträglichkeit und geringe Toxizität aus. Außerdem besitzen sie gute Wasserlöslichkeit. Die Wasserlöslichkeit ist pH-abhängig. Bei pH 9,0 besitzt das Rosiglitazon-Cholinat eine Wasserlöslichkeit, von 20,0 mg/ml, das Rosiglitazon-Lysinat eine solche von 9,4 mg/ml, während die des Rosiglitazon-Maleats nur 5,9 mg/ml und die des Rösiglitazon-Phosphats sogar nur 2,4 mg/ml beträgt. Bei pH 6,5 ist die Wasserlöslichkeit des Rosiglitazon-Cholinats mit 11,7 mg/ml um mehr als das Hundertfache höher als die des Rosiglitazon-Maleats (< 0.1 mg/ml). Überraschenderweise sind die erfindungsgemäßen Salze praktisch nicht hygroskopisch und haben eine ausgezeichnete Stabilität. Soweit die Erfindung vorliegend für Rosiglitazon beschrieben wird, gilt die Erfindung gleichermaßen für die Enantiomere und für tautomere Formen des Rosiglitazons.

Besonders bevorzugt wird derzeitig das Salz des Rosiglitazons mit Cholin.

Cholin ist eine wichtige Komponente in zahlreichen Stoffwechselfunktionen und findet als Therapeutikum Anwendung. Ferner ist es Bestandteil von Multivitaminpräparaten und in vielen Nahrungsmitteln enthalten. In üblichen Mengen eingenommen ist es praktisch nicht toxisch und daher sehr gut verträglich.

Lysin ist eine essentielle Aminosäure und in nahezu allen Eiweißen vorhanden. Seine pharmazeutische Verträglichkeit ist vielfach erprobt. Lysin ist als Nahrungsmittelzusatz besonders bei diätetischen Lebensmitteln in Gebrauch.

Arginin ist eine nicht-essentielle Aminosäure, die ebenfalls in nahezu allen Eiweißen vorkommt. Es wird sowohl als Nahrungsmittelzusatz als auch als Bestandteil von Therapeutika eingesetzt.

Die Aminosäuresalze können durch Lösen der Rosiglitazon-Base in siedendem Ethanol oder Methanol und Zugabe der Aminosäure als Feststoff oder in Lösung in warmem Wasser leicht hergestellt werden. Das Rosiglitazon-Cholinat kann auch zweckmäßig durch Vorlegen einer Suspension von Rosiglitazon in getrocknetem Ethanol, Versetzen mit einer Cholinlösung und Ausfällung des Salzes mit Essigester und Diethylether erhalten werden. Das Produkt fällt kristallin aus und wird bevorzugt bei 0°C abfiltriert.

Die erfindungsgemäßen Salze können auf an sich bekannte Art und Weise zu Arzneimitteln für Säuger, bevorzugt Menschen, formuliert werden. In den Arzneimitteln liegen die erfindungsgemäßen Salze im Gemisch mit einem pharmazeutischen organischen oder anorganischen Träger, der für enterale oder parenterale Verabreichungen geeignet ist, vor. Die orale Verabreichung der erfindungsgemäßen Salze über Tabletten, Kapseln, Pulver oder in flüssiger Form, wie als Suspensionen, in Lösung, als Emulsion oder als Sirup ist besonders bevorzugt.

Bei der Formulierung als Tabletten werden übliche Arzneimittelträger wie Natriumcitrat, Lactose, mikrokristalline Cellulase und Stärke, Schmiermittel wie wasserfreie Kieselsäure, hydriertes Castoröl, Magnesiumstearat, Natriumlaurylsulfat und Talk, sowie Bindemittel wie Stärkepaste, Glucose, Lactose, Gummi-Arabicum, Mannit, Magnesiumtrisilicat und Talk verwendet. Wenn die erfindungsgemäßen Salze über Flüssigkeiten verabreicht werden sollen, können übliche flüssige Träger verwendet werden.

Bevorzugt ist ebenfalls eine Formulierung für Injektionen und Infusionen, wie es auf dem Fachgebiet bekannt und in einschlägigen Standardwerken beschrieben ist.

Die erfindungsgemäßen Salze können ebenfalls als auf an sich bekannte Art und Weise als Depotformulierungen oder zu Arzneimitteln mit verzögerter oder hinhaltender Freisetzung formuliert werden.

### Beispiele

### Beispiel 1

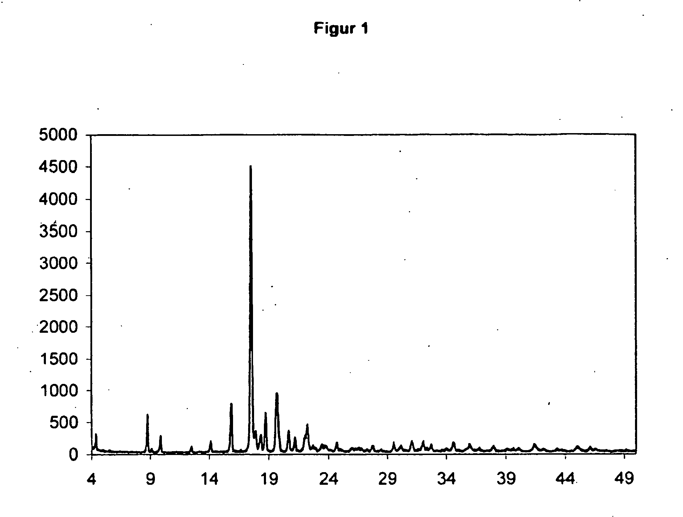

3,5 g Rosiglitazon wurden in 15 mL THF bei einer Badtemperatur von 50°C gelöst. 2,65 g 45%-ige methanolische Cholinhydroxidlösung wurden zu der Lösung gegeben. Nach 5 min Rühren wurden 75 mL Essigester nach und nach unter Rühren zugegeben. Zu der leicht trüben Lösung wurden Impfkristalle zugefügt und das Bad entfernt. Der Ansatz blieb über Nacht bei Raumtemperatur stehen. Das Produkt fiel in Form feiner, weißer Nadeln aus. Die Kristalle wurden durch Vakuumfiltration isoliert, mit 10 mL einer Mischung aus THF/Essigester 1:3 gewaschen und 24 h im Vakuum getrocknet
Ausbeute: 3 g
Schmpkt.: 101,5-103,8°C
¹H-Spektrum: Zusammensetzung Cholin/Rosiglitazon 1:1, praktisch kein Lösungsmittel sichtbar
Von dem Produkt wurde ein Pulverröntgenspektrum aufgenommen, das in Fig. 1 gezeigt ist. Auf der X-Achse ist der 2 θ-Wert aufgetragen, auf der Y-Achse die Intensität. Die erhaltene polymorphe Form ist durch die Hauptpeaks bei 2 θ von 8,76, 15,90, 17,59, 18,75, 19,73 und 22,24 gekennzeichnet, insbesondere durch folgende Peakliste:

### Pulver-Röntgenstrahlen-Diffraktion

| **Peakposition 2 θ (°)** | **Peakintensität** |
|---|---|
| 8,76 | 571,3 |
| 9,89 | 283,3 |
| 12,49 | 119,9 |
| 14,12 | 206,7 |
| 15,90 | 804,3 |
| 17,59 | 4529,8 |
| 17,92 | 319,2 |
| 18,34 | 290,5 |
| 18,75 | 669,7 |
| 19,73 | 952,5 |
| 20,72 | 369, 8 |
| 21,22 | 265,5 |
| 22.06 | 309,9 |
| 22,24 | 463,8 |
| 23,41 | 161,8 |
| 24,68 | 189,6 |
| 29,56 | 184,1 |
| 31,07 | 187,3 |
| 32,01 | 201,0 |
| 34,57 | 184,4 |

Die Messung erfolgte auf übliche Art und Weise mit Standardmethoden bei Raumtemperatur und Normaldruck. Als Fehlerbereich für jeden 2 θ-Wert kann 0,2 angegeben werden.

### Beispiel 2

5 g Rosiglitazon wurden in 25 mL trockenem Ethanol als Suspension vorgelegt, bei Raumtemperatur 3,87 g Cholinlösung dazu gegeben, 10 min gerührt und schließlich filtriert. Zum Filtrat wurden 100 mL Essigester und 100 mL Diethylether gegeben. Danach wurde über Nacht in den Kühlschrank bei 5°C gestellt. Das Produkt fiel in Form feiner, weißer Nadeln an. Die Kristalle wurden durch Vakuumfiltration isoliert und mit 10 mL Dietyhlether gewaschen. Das Produkt wurde bei Raumtemperatur 5 Tage lang bei 20 mbar getrocknet. Ausbeute: 4,5 g (70%)

### Beispiel 3

4 g Rosiglitazon wurden in 130 mL trockenem Ethanol in der Siedehitze gelöst. 1,642 g Lysin wurden in 5 mL warmem Wasser gelöst und zur heißen Lösung des Rosiglitazons gegeben. Es entstand eine klare Lösung, die 5 min zum Sieden erhitzt wurde. Das Wärmebad wurde entfernt und der Ansatz blieb bei Raumtemperatur stehen. Nach 3 h Stehen bei Raumtemperatur wurde über Nacht bei 5°C in den Kühlschrank gestellt. Das Produkt wurde durch Vakuumfiltration isoliert, mit 20 mL Ethanol gewaschen und im Vakuum mehrere Tage bei 50°C getrocknet. Ausbeute: 5,4 g

### Beispiel 4

1 g Rosiglitazon und 411 mg Lysin wurden in 10 mL siedendem Methanol (getrocknet) gelöst und in der Siedehitze 12 mL Isopropanol zugegeben. Nach ca. 5 min wurde das Heizbad entfernt und der Ansatz blieb über Nacht bei Raumtemperatur stehen. Das Produkt wurde durch Vakuumfiltration isoliert, mit Isopropanol und Dietyhlether gewaschen, dann im Vakuum 16 h bei 50°C getrocknet.
¹H-Spektrum: Zusammensetzung Lysin/Rosiglitazon 1:1, ca. 6 mol% Isopropanol

### Beispiel 5

1,05 g Rosiglitazon und 426 mg Lysin wurden in 10 mL siedendem Methanol (getrocknet) gelöst und in der Siedehitze 18 mL Essigester zugegeben. Nach ca. 5 min wurde das Heizbad entfernt und der Ansatz blieb über Nacht bei Raumtemperatur stehen. Das Produkt wurde durch Vakuumfiltration isoliert, mit Essigester gewaschen und 16 h im Vakuum bei 50°C getrocknet.
Ausbeute: 1 g
¹H-Spektrum: Zusammensetzung Lysin/Rosiglitazon 1:1, ca. 8 mol% Ethanol

### Beispiel 6

2 g Rosiglitazon und 976 mg Arginin wurden in 70 mL Ethanol ½h zum Sieden erhitzt. Die entstandene Lösung blieb über Nacht bei Raumtemperatur stehen. Das Produkt Wurde durch Vakuumfiltration isoliert, mit Ethanol gewaschen und 2 Tage im Vakuum bei 50°C getrocknet.
Ausbeute: 2,4 g

### Beispiel 7

Die Löslichkeit der in den Beispielen 1 und 3 hergestellten Rosiglitazon-Salze wurde bestimmt und mit der Löslichkeit des Maleatsalzes und der freien Base verglichen.

Folgende Pufferlösungen wurden verwendet:

| | |
|---|---|
| pH 1,5 | 2% Phosphorsäure |
| pH 3.0 | 188 mg KH2PO4 in 200 mL aqua purificata gelöst und mit 1,0n HCl eingestellt |
| pH 8,9 | 188 mg KH2PO4 in 200 mL aqua purificata gelöst und mit 1.0n NaOH eingestellt |
| pH 12,0 | 250 mg K2HO4 in 200 mL aqua purificata gelöst und mit 1,0n HCl eingestellt |

Es wurden jeweils ca. 100 mg der Untersuchungssubstanz in 10 mL (bei guter Löslichkeit wie z.B. pH 11,8 entsprechend weniger) der entsprechenden o.g. Pufferlösungen versetzt und mittels 1,0n HCl bzw. 1,0n NaOH auf den entsprechenden pH-Wert gebracht. Anschließend wurden die Suspensionen eine Minute im Ultraschallbad behandelt, der pH-Wert überprüft und gegebenenfalls erneut auf den jeweils unten angegebenen Wert eingestellt. Danach wurden die Lösungen 0.45µm-filtriert.

Bei den so hergestellten Lösungen wurde die Absorption bei 345 nm gemessen. Die Untersuchungslösungen wurden zur Messung mit Phosphorsäure einheitlich auf einen pH-Wert von 1,5 eingestellt. Als Kalibriersubstanz diente die freie Base.

Das Ergebnis ist in Tabelle 1 zusammengefasst. Daraus geht hervor, dass die Aminosäuresalze vor allem im physiologischen pH-Bereich eine deutlich bessere Löslichkeit aufweisen als die Base und das Maleatsalz, insbesondere zeigt das Cholinat bei pH 6,5 eine mehr als hundertfach höhere Löslichkeit.

**Tabelle 1: Löslichkeit der Aminosäuresalze von Rosiglitazon im Vergleich zum Maleatsalz und der freien Base.**

| **Löslichkeit bei** | **Cholinat** | **Lysinat** | **Maleat** | **Base** |
|---|---|---|---|---|
| **pH 4,6** | 1,1 mg/ml | 1,2 mg/ml | 0,6 mg/ml | 0,2 mg/ml |
| **pH 6,5** | 11,7 mg/ml | 0,2 mg/ml | < 0,1 mg/ml | 0,1 mg/ml |
| **pH 9,0** | 20,0 mg/ml | 9,4 mg/ml | 5,9 mg/ml | 5,9 mg/ml |
| **pH 11,8** | > 50 mg/ml | > 50 mg/ml | 19,2 mg/ml | > 50 mg/ml |

## Patentansprüche

1. Aminosäuresalze der racemischen oder einer enantiomeren oder tautomeren Form von Rosiglitazon (5-(4-(2-(N-methyl-N-(2-pyridyl)amino)ethoxy)benzyl)-2,4-thiazolidinedion) und die Solvate dieser Salze.

2. Aminosäuresalz nach Anspruch 1, nämlich Rosiglitazon-Cholinat und dessen Solvate.

3. Aminosäuresalz nach Anspruch 1, nämlich Rosiglitazon-Lysinat und dessen Solvate.

4. Aminosäuresalz nach Anspruch 1, nämlich Rosiglitazon-Arginat und dessen Solvate.

5. Polymorphe Form des Rosiglitazon-Cholinats, **gekennzeichnet durch** ein Pulverröntgendiffraktogram mit 2 θ-Werten bei 8,76, 15,90, 17,59, 18,75, 19,73 und 22,24.

6. Arzneimittel umfassend ein Aminosäuresalz oder Solvat nach einem der Ansprüche 1-5 und gegebenenfalls einen oder mehrere pharmazeutisch verträgliche Träger und/oder Hilfsstoffe.

7. Verwendung eines Aminosäuresalzes oder Solvats nach einem der Ansprüche 1-5 zur Herstellung eines Arzneimittels zur Behandlung oder Prophylaxe von Hyperglykämie, insbesondere Typ II-Diabetes, Hyperlipämie, Hochdruck, kardiovaskulären Erkrankungen und/oder Essstörungen.

8. Verfahren zur Herstellung eines Aminosäuresalzes oder Solvats nach einem der Ansprüche 1-5, **dadurch gekennzeichnet, dass** man das Racemat oder eine enantiomere oder tautomere Form des Rosiglitazons mit einer Aminosäure umsetzt, oder ein Salz des Rosiglitazons mit einer Aminosäure umsalzt.

## Claims

1. Amino acid salts of the racemic or an enantiomeric or tautomeric form of rosiglitazone (5-(4-(2-(N-methyl-N-(2-pyridyl)amino)ethoxy)benzyl)-2,4-thiazolidinedione) and the solvates of said salts.

2. Amino acid salt according to claim 1, namely rosiglitazone cholinate and the solvates thereof.

3. Amino acid salt according to claim 1, namely rosiglitazone lysinate and the solvates thereof.

4. Amino acid salt according to claim 1, namely rosiglitazone arginate and the solvates thereof.

5. A polymorphous form of the rosiglitazone cholinate, **characterized by** a powder X-ray diffractogram with 2 θ values at 8.76, 15.90, 17.59, 18.75, 19.73 and 22.24.

6. A medicament comprising an amino acid salt or solvate according to any of claims 1-5 and, where appropriate, one or more pharmaceutically compatible carriers and/or excipients.

7. Use of an amino acid salt or solvate according to any of claims 1-5 for the production of a medicament for treating or preventing hyperglycemia, in particular type II diabetes, hyperlipemia, high blood pressure, cardiovascular diseases and/or eating disorders.

8. A process for producing an amino acid salt or solvate according to any of claims 1-5, **characterized by** reacting the racemate or an enantiomeric or tautomeric form of rosiglitazone with an amino acid or converting a salt of rosiglitazone with an amino acid into another salt.

## Revendications

1. Sels d'acide aminé de la forme racémique ou d'une forme énantiomère ou tautomère de la rosiglitazone (5-(4-(2-(N-méthyl-N-(2-pyridyl)amino)éthoxy)benzyl)-2,4-thiazolidine-dione) et les solvates de ces sels.

2. Sel d'acide aminé selon la revendication 1, à savoir le cholinate de rosiglitazone et ses solvates.

3. Sel d'acide aminé selon la revendication 1, à savoir le lysinate de rosiglitazone et ses solvates.

4. Sel d'acide aminé selon la revendication 1, à savoir l'arginate de rosiglitazone et ses solvates.

5. Forme polymorphe du cholinate de rosiglitazone, **caractérisée par** un diffractogramme aux rayons X de poudre, ayant des valeurs 2θ à 8,76, 15,90, 17,59, 18,75, 19,73 et 22,24.

6. Agent pharmaceutique comprenant un sel d'acide aminé ou un solvate selon l'une quelconque des revendications 1-5 et le cas échéant, un ou plusieurs supports et/ou auxiliaires pharmaceutiquement compatibles.

7. Utilisation d' un sel d' acide aminé ou d' un solvate selon l'une quelconque des revendications 1-5, pour la préparation d'un agent pharmaceutique pour le traitement ou la prophylaxie de l'hyperglycémie, en particulier le diabète de type II, l'hyperlipidémie, l'hypertension, des maladies cardiovasculaires et/ou des troubles de 5l'alimentation.

8. Procédé de préparation d'un sel d'acide aminé ou un d'solvate selon l'une quelconque des revendications 1-5, **caractérisé en ce que** l'on fait réagir le racémique ou une forme énantiomère ou tautomère de la rosiglitazone avec un acide aminé, ou **en ce qu'**on modifie un sel de la rosiglitazone en un sel d'acide aminé.
